## Europäisches Patentamt

## European Patent Office

## Office européen des brevets

(11) Numéro de publication: **0 203 869**
**B1**

## (12) FASCICULE DE BREVET EUROPÉEN

(45) Date de publication du fascicule du brevet:
**02.11.88**

(51) Int. Cl.⁴: **C 12 M 1/00, C 12 N 13/00**

(21) Numéro de dépôt: **86401137.4**

(22) Date de dépôt: **29.05.86**

---

(54) **Dispositif à base d'une matière cristalline minérale relié à une source d'énergie et utilisation dudit dispositif pour améliorer le métabolisme bactérien.**

---

(30) Priorité: **30.05.85 FR 8508147**

(43) Date de publication de la demande:
**03.12.86 Bulletin 86/49**

(45) Mention de la délivrance du brevet:
**02.11.88 Bulletin 88/44**

(84) Etats contractants désignés:
**AT BE CH DE FR GB IT LI LU NL SE**

(56) Documents cité:
**FR-A-815 261
FR-A-843 994
FR-A-2 059 215
FR-A-2 222 433
GB-A-1 324 403
US-A-3 618 734**

(73) Titulaire: **MASSON, Alain, La Besserie, F-86130 Saint Cyr (FR)**

(72) Inventeur: **MASSON, Alain, La Besserie, F-86130 Saint Cyr (FR)**

(74) Mandataire: **Portal, Gérard, Cabinet Beau de Loménie 55, rue d'Amsterdam, F-75008 Paris (FR)**

## Description

La présente invention concerne un dispositif utilisable notamment pour améliorer le métabolisme bactérien.

On connaît déjà différents dispositifs permettant d'accélérer et d'augmenter la croissance de microorganismes (FR-A-2 222 433) de traiter des liquides (FR-A-2 059 215), ou d'activer et ranimer la fermentation alcoolique dans des liquides incomplètement fermentés, ou pour les stériliser (brevets français Nos 815 261 et 843 994).

Cependant, selon ces documents antérieurs, un champ électrique ou électromagnétique est développé directement dans le bouillon de culture ou le milieu de fermentation, et les électrodes ou les solénoïdes sont plongés dans le milieu liquide de fermentation ou de culture.

L'invention est basée sur une conception tout à fait originale, selon laquelle l'énergie est apportée à une masse pulvérulente séparée du milieu de culture.

En effet, selon l'invention, le dispositif est constitué d'un mélange homogène d'une matière minérale pulvérisée cristallisée en couches octaédriques et/ou tétraédriques et d'un matériau organique amorphe (non cristallin), ledit mélange étant enfermé dans une enveloppe inerte et l'ensemble étant relié à une source d'énergie.

Parmi les matières cristallines qui conviennent pour la présente invention, il a été trouvé que les phyllosilicates seuls ou en mélange étaient particulièrement avantageux. Les phyllosilicates présentent une structure lamellaire et sont constitués par des feuilles formées de tétraèdres et d'octaèdres. Parmi ces matières, on peut citer la kaolinite, la serpentine, le talc, le mica, le quartz. Mais il est évident que cette liste n'est pas limitative.

D'autres matières, comme l'amiante, peuvent également convenir.

En général, les diélectriques absolus conviennent aussi. La poudre minérale finement divisée est mélangée, de manière homogène de préférence, avec un matériau amorphe ou non cristallin. Parmi ces matériaux, on choisira avantageusement les hydrates de carbone comme le glucose ou ses analogues.

L'ensemble est enveloppé partiellement ou totalement par une enveloppe de maintien inerte, de préférence comme du carton, une feuille de matériau plastique ou le bois.

Parmi les sources d'énergie, on emploiera avantageusement l'énergie électrique, sinusoïdale (courant alternatif) ou continu. On pourra également utiliser une autre forme d'énergie appropriée.

Ainsi, un mode de mise en oeuvre avantageux réside en un dispositif caractérisé en ce que la source d'énergie électrique est constituée par un filament noyé dans le mélange et relié à une source de courant alternatif ou continu.

De préférence, l'intensité est comprise entre 0,1 mA et 10 A, la tension entre 0,1 et 100 V et, dans le cas du courant alternatif, la fréquence entre 0,1 Hz et $2.10^9$ Hz. De préférence, on choisit une intensité entre 0,1 et 1 A, une tension entre 1 et 10 V et une fréquence entre 0,1 et 10 MHz. Il a été trouvé que le dispositif décrit ci-dessus permettait d'accroître sensiblement la durée de vie des bactéries d'un milieu de culture lorsqu'il était placé à proximité de ce milieu dans une étuve permettant de conserver l'atmosphère appropriée au développement des organismes.

L'utilisation du dispositif selon l'invention n'est pas limitée à une famille de bactéries particulières et elle peut convenir à toutes les bactéries.

Le milieu de culture permettant à des bactéries de se développer est bien connu de tous les microbiologistes. Rappelons simplement que ces organismes se développent dans un milieu aqueux tamponné dont le pH est voisin de 7, à température voisine de 37°C, en présence notamment d'une source d'hydrate de carbone.

Avec le dispositif selon l'invention, on observe une augmentation considérable de la population bactérienne vivante en un temps très court (moins de 72 h).

Bien entendu, le dispositif n'est pas limité à cette application, mais est revendiqué en tant que tel.

L'invention sera mieux comprise à l'aide d'un exemple particulier de réalisation qui doit être considéré comme purement illustratif et ne limitant en rien l'invention.

La figure 1 est une vue en coupe longitudinale du dispositif.

La figure 2 est une vue en coupe longitudinale du dispositif en cours d'utilisation.

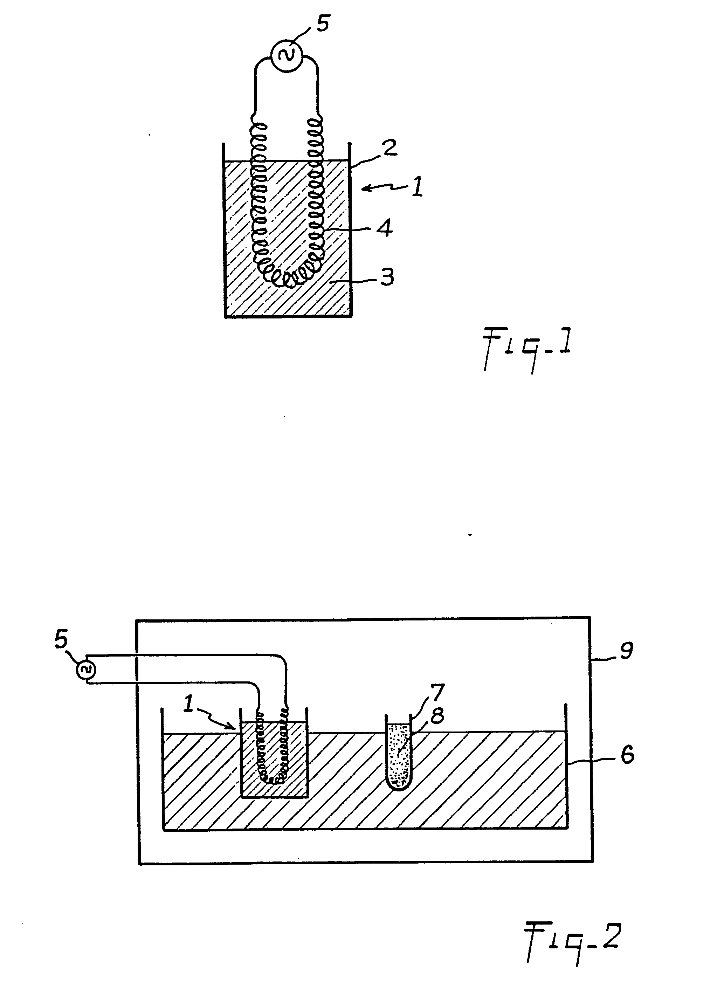

Selon la figure 1, le dispositif 1 est constitué d'une enveloppe cylindrique 2 ouverte à son sommet, en carton, contenant jusqu'à une certaine hauteur le mélange 3 de poudre suivant :

14,6 % en poids de kaolinite
72,2 % en poids de talc
13,2 % en poids de glucose.

Un filament 4 en spirale est noyé dans la poudre et est relié à une source de courant alternatif 5. Cette enveloppe 2 est elle-même enfoncée dans un bac 6 en matière quelconque qui contient du mélange 3 et dans lequel est enfoncé un tube à essai 7 contenant le milieu de culture 8. Le bac 6 est lui-même placé dans une étuve 9.

Les conditions d'utilisation sont les suivantes:

intensité                    500 mA

2

| tension alternative | 5 V |
| fréquence | 1 MHz |

Le dispositif selon l'invention offre également la possibilité de conditionner des liquides aqueux ou organiques en plaçant le liquide à proximité du dispositif.

L'invention est maintenant illustrée par des exemples de réalisation effectués avec différentes sortes de bactéries.

## Exemple 1

La souche bactérienne est Haemophilus influenzae CNCM S2/52 sérotype b.

Elle est entretenue par réensemencements successifs en milieu liquide ou solide approprié et conservée en milieu liquide glycériné, congelée à -80°C.

Le milieu de culture est un bouillon Eugon tamponné à pH 7,6 avant stérilisation, supplémenté en facteurs de croissance. Le facteur V est apporté par une solution de β Nicotinamide adénine dinucléotide (Sigma) à 0,2 % (p/v) stérilisée par filtration sur membrane, ajoutée à raison de 1 % (v/v). Le facteur X est apporté par une solution d'hémoglobine (Prolabo) à 2 % (p/v), précipitée et stérilisée à l'autoclave (15 min à 120°C) ajoutée à raison de 5 % (v/v).

Le milieu liquide est stérilisé par autoclave 15 min à 120°C. Le milieu de numération est une gélose chocolat (Bio. Mérieux) additionnée du liquide d'enrichissement Polyvitex.

La température des tubes est de 37 ± 0,5°C. L'ensemencement du bouillon de préculture est réalisé à raison de 5 % dans des tubes 18 x 180 contenant 20 ml de milieu de culture. Ce volume ensemencé est ensuite partagé en deux fractions égales, de façon que les contenus des deux tubes participant à l'essai soient au maximum identiques.

La mise en incubation est réalisée très rapidement dans l'étuve dont la température est stabilisée depuis au moins 24 h.

Après 24, 48, éventuellement 72 h, 7 jours et 14 jours, 1 ml de chaque tube est prélevé pour les numérations.

La numération est réalisée en surface. Le nombre est ramené au ml.

L'essai a été répété 15 fois avec numération aux temps t = 0, t = 24 h, t = 48 h. Les résultats sont reportés sur les tableaux I et II. Le tableau I correspond aux valeurs moyennes obtenues en fonction du temps quelle que soit la valeur de l'inoculum ; le tableau II tient compte de la valeur de l'inoculum, les différentes valeurs de celui-ci étant réparties en classes.

Chaque fois, des mesures sont faites avec l'un des tubes à essai non soumis à l'action du dispositif.

## Tableau I:

Haemophilus influenzae: nombre de germes vivants après 24 et 48 h d'incubation (moyenne de 15 essais et intervalle de confiance au risque de 5 %)

Culture

| Temps d'incubation | Normale | Modifiée par le dispositif |
|---|---|---|
| 24 h | $1,3 + 0,6.10^8$ | $1,9 + 0,2.10^8$ |
| 48 h | $3,4 + 1,3.10^6$ | $31,4 + 10,5.10^6$ |

3

**Tableau II :**

Haemophilus influenzae : nombre de germes vivants après 24 et 48 h d'incubation en atmosphère normale et modifiée selon l'importance de l'inoculum

Temps d'incubation

| Germes au t = 0 $\times 10^6$ | 24 h | | 48 h | |
|---|---|---|---|---|
| | Culture | | | |
| | normale $\times 10^8$ | modifiée $\times 10^8$ | normale $\times 10^6$ | modifiée $\times 10^6$ |
| 0,87 | 1,2 | 1,63 | 3,60 | 15,00 |
| 1,15 | 1,25 | 1,54 | 3,70 | 14,50 |
| 1,42 | 1,16 | 1,58 | 4,00 | 16,00 |
| 1,50 | 1,40 | 2,80 | 0,69 | 23,00 |
| 1,65 | 1,20 | 1,65 | 3,10 | 13,00 |
| 2,44 | 0,50 | 1,84 | 1,18 | 18,00 |
| 3,23 | 0,63 | 2,14 | 1,13 | 21,00 |
| 4 | 0,76 | 1,60 | 0,82 | 17,00 |
| 4,43 | 0,79 | 1,90 | 8,60 | 63,00 |
| 4,9 | 1,60 | 2,40 | 6,50 | 52,00 |
| 6,2 | 4,90 | 2,50 | 0,60 | 13,00 |
| 6,6 | 0,32 | 1,40 | 3,40 | 52,00 |
| 8,6 | 1,55 | 1,80 | 4,50 | 55,00 |
| 12,6 | 0,52 | 1,60 | 2,70 | 46,00 |
| 14 | 1,70 | 2,10 | 6,50 | 52,00 |

**Exemple 2**

La souche bactérienne est Listeria monocytogènes CNCM 6089 sérotype 4.

Le traitement de cette souche est identique à celui de l'exemple 1, mis à part le fait que le bouillon Eugon est tamponné à pH 7,2 avant stérilisation et que le milieu de numération est une gélose caséine soja (Merck) additionnée de glucose à raison de 5 % (p/v).

La numération est en outre réalisée en profondeur. Les résultats sont indiqués dans le tableau III ci-après.

**Tableau III :**

Listeria monocytogenes : nombre de germes vivants après 24, 48 et 72 h d'incubation en atmosphère normale et modifiée (moyenne de trois essais sur 3 tubes et intervalle de confiance au risque de 5 %)

Temps d'incubation

| Essai n° | 24 h | | 48 h | | 72 h | |
|---|---|---|---|---|---|---|
| | Culture | | | | | |
| | normale | modifiée | normale | modifiée | normale | modifiée |
| I | $1{,}25.10^9$ | $1{,}30.10^9$ | $1{,}2.10^8$ | $4{,}8.10^8$ | $0{,}40.10^6$ | $88.10^6$ |
| II | $0{,}40.10^9$ | $1{,}20.10^9$ | $0{,}2.10^8$ | $2{,}0.10^8$ | $0{,}87.10^6$ | $35.10^6$ |
| III | $0{,}70.10^9$ | $1{,}25.10^9$ | $1{,}9.10^8$ | $4{,}2.10^8$ | $1{,}12.10^6$ | $10{,}3.10^6$ |
| moyenne | $0{,}79.10^9$ | $1{,}25.10^9$ | $1{,}09.10^8$ | $3{,}7.10^8$ | $0{,}80.10^6$ | $15.10^6$ |
| intervalle de con-fiance (5 %) | $0{,}40.10^9$ | $0{,}06.10^9$ | $0{,}70.10^8$ | $1{,}3.10^8$ | $0{,}33.10^6$ | $32.10^6$ |

**Exemple 3**

La souche bactérienne est Escherichia Coli CNCM 54127.

Le traitement de la souche est identique à celui de l'exemple 1, mis à part le fait que le bouillon est le Trypticase Soja (Bio. Mérieux) pour les précultures et un bouillon Protéose-peptone n° 3 (Difco) pour les cultures et que le milieu de numération est une gélose caséine soja (Merck) additionnée de glucose à raison de 5 % (p/v).

La numération est réalisée en profondeur. Les résultats sont réunis dans le tableau IV ci-après.

**Tableau IV**

<u>Escherichia Coli :</u> nombre de germes vivants après 24, 72 h, 7 et 14 jours d'incubation en atmosphère normale et modifiée (moyenne de 3 essais sur 2 tubes et intervalle de confiance au risque de 5 %).

Temps d'incubation

| Essai n° | 24 h culture normale | 24 h culture modifiée | 72 h culture normale | 72 h culture modifiée | 7 jours culture normale | 7 jours culture modifiée | 14 jours culture normale | 14 jours culture modifiée |
|---|---|---|---|---|---|---|---|---|
| I | $1,6 \cdot 10^9$ | $2,2.10^9$ | $0,8.10^9$ | $0,6.10^9$ | $1,1 \cdot 10^8$ | $2,7.10^8$ | $2.10^7$ | $4,7.10^7$ |
| II | $2,1 \cdot 10^9$ | $2,3.10^9$ | $0,7.10^9$ | $0,7.10^9$ | $0,8 \cdot 10^8$ | $3,2.10^8$ | $0,7.10^7$ | $8,5.10^7$ |
| III | $0,99.10^9$ | $0,9.10^9$ | $0,7.10^9$ | $0,5.10^9$ | $0,9 \cdot 10^8$ | $5,6.10^8$ | $3,3.10^7$ | $9.10^7$ |
| moyenne | $1,5 \cdot 10^9$ | $1,8.10^9$ | $0,7.10^9$ | $0,6.10^9$ | $0,98.10^8$ | $3,8.10^8$ | $2.10^7$ | $7,4.10^7$ |
| intervalle de confiance | $0,6 \cdot 10^9$ | $0,7.10^9$ | $0,1.10^9$ | $0,2.10^9$ | $0,3 \cdot 10^8$ | $1,6.10^8$ | $2.10^7$ | $3,5.10^7$ |
| ($\alpha = 5$ %) | NS | | NS | | $p < 1$ ‰ | | $\rho = 1$ % | |

**Revendications**

1. Dispositif (1) constitué d'un mélange (3) homogène d'une matière minérale pulvérisée cristallisée en couches octaédriques et/ou tétraédriques et d'un matériau organique amorphe (non cristallin) ledit mélange étant enfermé dans une enveloppe inerte (2) et l'ensemble étant relié à une source d'énergie (5).

2. Dispositif selon la revendication 1, caractérisé en ce que la matière cristalline minérale est un phyllosilicate.

3. Dispositif selon la revendication 1, caractérisé en ce que le matériau est un hydrate de carbone.

4. Dispositif selon la revendication 1, caractérisé en ce que l'enveloppe inerte est constituée de carton, d'une feuille de matière plastique ou de bois.

5. Dispositif selon la revendication 1, caractérisé en ce que l'ensemble est relié à une source d'énergie électrique.

6. Dispositif selon la revendication 5, caractérisé en ce que la source d'énergie électrique est constituée par un filament (4) noyé dans le mélange (3) relié à une source de courant alternatif (5) ou continu.

7. Utilisation d'un dispositif selon l'une des revendications précédentes pour améliorer le métabolisme bactérien, caractérisée en ce qu'on met ledit dispositif à proximité d'un milieu de culture.

8. Utilisation selon la revendication 7, caractérisée en ce que dans le cas où la source d'énergie électrique est constituée par un filament noyé dans le mélange, relié à une source de courant alternatif ou continu, l'intensité dudit courant est comprise entre 0,1 mA et 10 A, la tension entre 0,1 et 100 volts, et, dans le cas du courant alternatif, la fréquence entre 0,1 Hz et $2.10^9$ Hz.

**Patentansprüche**

1. Vorrichtung (1), bestehend aus einer homogenen Mischung (3) eines kristallinen, pulverformigen mineralischen Materials aus Oktaeder- und/oder Tetraederlagen und eines (nicht kristallinen) amorphen organischen Materials, wobei die Mischung in einer inerten Hülle (2) eingeschlossen und die Gesamtheit mit einer Energiequelle (5) verbunden ist.

2. Vorrichtung nach Anspruch 1, dadurch gekennzeichnet, daß das kristalline mineralische Material ein Phyllosilikat ist.

3. Vorrichtung nach Anspruch 1, dadurch gekennzeichnet, daß das Material ein Kohlenwasserstoffhydrat ist.

4. Vorrichtung nach Anspruch 1, dadurch gekennzeichnet, daß die inerte Hülle aus Karton, aus einer Plastikfolie oder aus Holz gebildet ist.

5. Vorrichtung nach Anspruch 1, dadurch gekennzeichnet, daß die Gesamtheit mit einer elektrischen Energiequelle verbunden ist.

6. Vorrichtung nach Anspruch 5, dadurch gekennzeichnet, daß die elektrische Energiequelle durch einen Faden (4) gebildet ist, der in der Mischung versenkt und an eine Quelle von Wechselstrom (5) oder Gleichstrom angeschlossen ist.

7. Verwendung einer Vorrichtung nach einem der vorhergehenden Ansprüche zur Verbesserung des Bakterienmetabolismus, dadurch gekennzeichnet, daß man die Vorrichtung in die Nähe eines Kulturmediums stellt.

8. Verwendung nach Anspruch 7, dadurch gekennzeichnet, daß für den Fall, daß die elektrische Energiequelle durch einen in die Mischung versenkten, an eine Quelle von Wechselstrom oder Gleichstrom angeschlossenen Faden gebildet ist, die Stromstärke zwischen 0,1 mA und 10 A, die Spannung zwischen 0,1 und 100 Volt und, bei Wechselstrom, die Frequenz zwischen 0,1 Hz und $2.10^9$ Hz liegt.

**Claims**

1. Device (1) constituted of a homogeneous mixture (3) of a crystallized pulverized mineral material in octahedral and/or tetrahedral layers, and of an amorphous organic material (non-crystallin), said mixture being contained in an inert envelope (2) and the whole being connected to a source of power (5).

2. Device according to claim 1, characterized in that the mineral crystallin material is a phylosilicate.

3. Device according to claim 1, characterized in that the material is a carbon hydrate.

4. Device according to claim 1, characterized in that the inert envelope is constituted of cardboard, of a sheet of plastic material or of wood.

5. Device according to claim 1, characterized in that the whole is connected to a source of electrical power.

6. Device according to claim 5, characterized in that the source of electrical power is constituted by a filament (4) embedded in the mixture (3) and connected to an A.C. or D.C. source (5).

7. Use of a device according to one of the preceding claims, for improving the bacterial metabolism, characterized in that said device is placed close to a culture medium.

8. Use according to claim 7, characterized in that in the case where the source of electrical power is constituted by a filament embedded in the mixture, and connected to an A.C. or D.C. source, the intensity of said current ranges between 0.1 mA and 10A, the voltage between 0.1 and 100 volts, and in the case of A.C. voltage, the frequency ranges between 0.1 Hz and $2.10^9$ Hz.

Fig-1

Fig-2